# EUROPEAN PATENT APPLICATION

(11) **EP 2 258 216 A1**
(43) Date of publication of application: **08.12.2010**
(21) Application number: 09161667.2
(22) Date of filing: 02.06.2009
(51) Int. Cl.: A23L 1/29, A23L 1/30

(54) **Nutritional Composition for Supporting Brain Development and Function of Toddlers**

(71) Applicant: Nestec S.A., 1800 Vevey (CH)
(72) Inventor: Biehl, Jan, CH-3600 Thun, (CH); Destaillats, Frédéric, CH-1077 Servion, (CH); Fay, Laurent, F-74500 Evian, (FR); Fukushima, Yoichi,, Saitama 331-0805, (JP); Schmitt, Jeroen Antonius Johannes,, CH-1510 Moudon, (CH); Wang, Bing, Tianjin 300042, (CN)
(74) Representative: Chautard, Cécile

(57) **Abstract**

The present invention relates to a nutritional composition, in particular directed to toddlers and/or a weaning child, said nutritional composition comprising a protein source, a source of available carbohydrates, a lipid source, at least one probiotic microorganism, and prebiotics, wherein said lipid source comprises DHA (docosahexaenoic acid). The nutritional composition improves cognitive performance, in particular learning and memory of the child. Preferably, the composition comprises iron, zinc, vitamin D and/or sialic acid. Preferably, the composition comprises a source ofphospholipids rich in DHA.

## Description

### Technical Field

The present invention relates to a nutritional composition. Furthermore, the present invention relates to a nutritional composition for improving the cognitive performance of children, in particular children of an age of about 1 to 7, preferably about 1 to 3 years.

### Prior Art and the Problem Underlying the Invention

Human breast milk is the ideal form of nutrition for infant and offers medical and psychological benefits not available from human milk substitutes. WHO recommends that all babies should be breastfed exclusively for the first six months of life. Thereafter, the babies should received appropriate complementary feeding with continued breastfeeding up to two years or beyond (WHO Infant and young child feeding, 2009). In some situations, however, breast milk is not available or not the preferred option of providing nutrition to an infant. Infant formulas, generally based on cow's milk, can provide a satisfactory alternative to breast milk as a sole source of nutrient to young infants. In the period starting from the age of about 6 to 18 months, a child is generally weaning and starts to take up solid food. Instead of milk-based infant formula, pure milk will continue to make a major contribution of energy and important nutrient requirements to the children at this age.

Infants should not be given unmodified cow's milk as a drink before at least the age of 9 months, because cow's milk alone is not suitable to meet the increased needs of some nutrients, such as iron, for example. Follow-up formulae are designed to complement the changing diet of the older infant and provide a more balanced and complete food, better adapted to the child's nutritional needs at this age than normal milk. Growing-up milks (GUMs) can be considered a subgroup of follow-up formulas, adapted more particularly to the nutritional needs of children of one year or older, for example 1-6 years. Accordingly, the objective of follow-up formulae and growing-up milks has generally been to provide nutrients that are important for supporting the healthy growth of a child. Generally, GUMs are adapted specifically to the nutritional needs of children of a specific age. For example, there are GUMs for children of 1-3 years, 3-5 years and above 5 years old.

More recently, it has also become an objective to strengthen a child's immune defences. For example, in US2007/0031537, a formula comprising LC-PUFA (long-chain poly unsaturated fatty acids) and a probiotic is disclosed. However, the formula disclosed in this document is directed to infants, not to children above 1 year.

Brain development and growth exceeds that of any other organ or body tissue, reaching its peak at 26 weeks of gestation and continuing at a rapid rate throughout the first two to three years of life. The energy demands (kcal/g/min) of brain and other neuronal tissue are extremely high: approximately 16 times that of skeletal muscle. Brain energy demands in the foetus and newborn are >80% of resting energy expenditure (adult 20-25%). The large allocation of our energy budget to brain metabolism has important implications for our dietary need. Sub-optimal nutrition during this phase has well recognised, irreversible consequences for cognitive function. Also during this critical period, the molecular, biochemical, physiological, and psychological development are established, which influences events all the way into adulthood.

In addition to strengthening a child's immune defences and improving a child's health, physical well-being and tonus, the present invention addresses the problem of providing further benefits to children of the age of about 1 to 6, preferably 1 to 3 years.

In addition, the present invention has to objective to provide a nutritional composition for children of the indicated age, said nutritional composition supports normal brain and/or mental development of the child and the associated mental and/or cognitive aspects.

Furthermore, the present invention has to objective to provide a nutritional composition that contributes to a good performance of a child in school, to an adaptive behaviour, to cognitive function, to language skills, to motor skills and to socio-emotional skills.

For example, the present invention addresses the problem of providing nutritional composition that contributes to or even improves the normal mental performance, cognition, visual function, language abilities and intellectual development of a child.

The present invention also addresses the problem of providing a nutritional composition comprising nutrients and precursors that are important building blocks for newly laid down tissues during brain maturation. In other words, the invention provides a nutritional composition providing the right and/or necessary nutrition for the developing brain of a child. This implies nutrients that the child's body needs for building up the brain ("structural building blocks"), nutrients that are necessary for the processes, such as metabolic processes, taking place in the brain, and nutrients that provide an adequate availability of energy that the brain needs for proper functioning.

More specifically, the present invention addresses the problem of providing nutritional composition that contributes to or even improves mental capacities and properties such as short and long term memory, learning capacity, alertness, attentiveness and concentration capacity of a child.

It is important to note that the present invention addresses the above problems while at the same time providing a balanced nutrition, providing important macro- and micronutrients, such as proteins, carbohydrates and fats, and vitamins and minerals, respectively.

The present invention addresses the problems depicted above, which problems thus are integral part of this invention.

### Summary of Invention

The present invention provides a nutritional composition comprising a protein source, a source of available carbohydrates, a lipid source, wherein said lipid source comprises DHA (docosahexaenoic acid).

In another aspect, the present invention provides a nutritional composition comprising:
- one or more protein source,
- one or more source of available carbohydrates,
- one or more lipid source,
- one or more probiotic microorganism, and,
- prebiotics,
wherein at least one of said one or more lipid source comprises DHA (docosahexaenoic acid).

According to an aspect, DHA is provided in the form of a phospholipid rich in DHA, in particular phosphatidylcholine (PC) and/or PE (phosphatidylethanolamine) form.

According to an aspect, the present invention provides the use of a protein source, a source of available carbohydrates, a lipid source and other ingredients as disclosed in this specification for preparing a nutritional composition.

Remarkably, the nutritional composition as defined by the present invention and its preferred embodiments, if used as nutritional complement in children of 1 to 7, preferably 1 to 3 years, provides nutrients useful to contribute to the body's buildup of the brain and/or to its proper functioning.

Furthermore, the nutritional composition is useful as a growing up milk, contributes to or even improves the normal mental performance, cognitive, behavioural and visual function, language ability and intellectual development of a child.

Accordingly, the present invention provides, in an aspect, the use of the nutritional composition as disclosed herein for supporting and/or improving the verbal, perception, quantitative abilities, attention as well as memory and psychomotor development in a child of 1 to 7, preferably 1 to 3 years of age.

In a similar aspect, the present invention provides, in another aspect, the use of the nutritional composition as disclosed herein for supporting and/or improving behavioural, cognitive, communicational, such as language, expressive and receptive communication, motor and social-emotional skills in a child of 1 to 7, preferably 1 to 3 years of age.

According to an aspect, the present invention provides, in another aspect, the use of the nutritional composition as disclosed herein for supporting and/or improving early child development. Early child development refers, for example to comparable or better changes in the body weight, height and head circumference and the efficacy of grow up formula from the occurrence of diarrhea.

Further, aspects and preferred embodiments of the present invention are provided in the description below as well as in the appended claims.

### Detailed Description of the Preferred Embodiments

The nutritional composition preferably comprises a protein source, a source of available carbohydrates, and a lipid source. Preferably, the composition comprises at least one probiotic microorganism. Preferably, the nutritional composition comprises soluble fiber, in particular at least one prebiotic. The term "comprises", for the purpose of the present invention means "includes, amongst other" and is not intended to mean "consists only of".

In the present specification, the term "nutrient" is repeatedly used. The term "nutrient" refers to typical macro-and micronutrients, such as proteins, lipids, carbohydrates, vitamins, minerals and trace elements, for example. However, for the purpose of the present specification, the term nutrient may also be used with respect to ingredients that are added for the purpose of exerting any kind of beneficial purpose on the child, for example, non-essential but beneficial components such as, for example, probiotics, fiber, in particular prebiotics, LC-PUFA including DHA (docosahexaenoic acid) and preferably ARA (arachodonic acid) and other functional ingredients.

For the purpose of the present specification, a child of 1 year refers to a child of the age of at least 1 year and up to 2 years, but not yet having complete 2 years elapsed. Accordingly, an age range of 1-2, for example, refers to a spent life span of at least 1 year and up to 3 years, but not yet 3 complete years elapsed. A child having an age of 1 year, 11 months and 30 days is encompassed in the age group of 1-2 years.

Preferably, the nutrients provided in the nutritional composition are adapted to the needs and nutritional recommendations of children of a specific age.

According to an embodiment, the nutritional composition of the present invention is adapted, designed and/or made for children of an age of eight months to 7 years, in particular 1-7, preferably 1-6, more preferably 1-5, more preferably 1-4, even more preferably 1-3 and most preferably 1-2 years.

In a particular aspect of the invention, the nutritional composition is adapted and/or designed for children of an age of 3-7, preferably 3-6, more preferably 3-5, most preferably 3-4. For any age, some or all nutrients are preferably adapted to meet the specific needs of a child. Below, a nutritional composition for children of 1-3 years, in particular 1-2 years is disclosed, and further below for children of 3-7 years.

According to an embodiment of the nutritional composition of the invention, said protein source provides about 11 to about 18%, preferably about 12-16%, of the total energy, said source of available carbohydrates provides from about 46 to about 54%, preferably about 48-53% of the total energy, and said lipid source provides from about 31 to about 39%, preferably about 33-37.5% of the total energy of the composition.

The protein source may be of vegetal or animal origin, for example. Examples of protein sources soy protein, rice protein, wheat protein, milk protein and egg protein. Preferably, the nutritional composition comprises milk protein. Preferably, the composition comprises whey protein, casein, or both. More preferably, the composition comprises whey protein and casein. The protein source may be provided in the form of intact protein, partial and total protein hydrolysates, di-, tri-, oligo- and polypeptides, free amino acids, and mixtures of the aforementioned, for example.

Milk, for the purpose of the present invention, is generally obtained from domesticated animals, such as cow, goat, sheep, camel, dromedary, buffalo, and so forth. Preferably, milk is cow's or goat's milk. Accordingly, milk protein may be, for example, cow's milk protein. Milk contains several nutrients that are implied in the buildup and the functioning of the brain, thereby supporting cognitive development of a child. Without wishing to be bound by theory, the present inventors believe that by combining the ingredients naturally contained and delivered by milk, if combined with further nutrient in a nutritional composition, mutually interact and together synergistically affect brain buildup and functions as defined in greater detail further below.

The expression "available carbohydrates" refers to carbohydrates that break down during digestion, in particular in the upper digestive tract during the cephalic and intestinal phase, under the action of digestive enzymes produced by the human body, but also to carbohydrates that are directly absorbed, such as glucose. In particular, the available carbohydrates refer to a source of carbohydrate such as sugar (for example saccharose, glucose, fructose) and starch that can be digested by human enzymes. Generally, the available carbohydrates are absorbed and enter into the intermediary metabolism. Generally, available carbohydrates, if not directly provided in the form of glucose, are carbohydrates, which can be converted by the body to glucose, since the latter is the main source of energy used by the brain under normal physiological conditions.

According to a preferred embodiment, the nutritional composition is free of starch. Preferably, available carbohydrates are provided in the form of at least one selected from lactose, other sugars (in particular saccharose, glucose, and/or fructose) and maltodextrin, and preferably a combination of at least two or of all three of the latter. Preferably, maltodextrin provides at least 50%, more preferably at least 54% by dry weight of all available carbohydrates. Preferably, lactose provides less than 50%, more preferably less than 46% by dry weight of all available carbohydrates. Preferably, saccharose provides less than 2%, more preferably less than 1% by dry weight of all available carbohydrates.

The lipid source includes lipids useful form human nutrition of any origin, such as vegetal and animal oils or fat, for example. Lipid sources include lipids obtained from milk, eggs, seeds, nuts, fungi, microorganisms such as yeast and bacteria, for example. Lipid sources include sources containing phospholipids, mono- di-, triglycerides, fatty acids, sterol-related compounds such as cholesterol, sphingosines, ceramides, glycosphingolipids, gangluiosides and the like. Preferred sources of lipids are milk, vegetal oils and eggs.

Among the lipids, there are important elements that the human body uses in order to build up the brain, especially during infancy and childhood. Nervous tissue has the second highest concentration of fatty acids after adipose tissue. Fatty acids constitute slightly more than 50% of the dry weight of the brain. Levels of long chain polyunsaturated fatty acids are particularly high in the cerebral cortex and the retina. Brain phospholipids contain a high proportion of arachidonic acid (ARA, 20:4 ω-6). Brain is also unusually rich in ω-3 fatty acids, in particular docosahexaenoic acid (DHA, 22:6 ω-3, also referred to as cervonic acid). DHA can account for up to 50% of phospholipid fatty acid in the cerebral cortex and the retina, being particularly abundant in the rod photoreceptors and in gray matter. This suggests that DHA is heavily involved in neuronal and visual functions. In the brain, DHA and ARA can be synthesised from essential fatty acids, such as linoleic acid (LA: 18:2 ω-6) and α-linolenic acid (ALA: 18:3 ω-3). In summary, DHA is a key component of cell membranes in the brain and the retina, where it accumulates during development.

The brain accumulates DHA during childhood, so that by the age of 5-6 years, most of the brain growth is completed. It is thus important to provide lipids in a way that supports the brain development during this period. Lipids, for example DHA, are preferably provided in a way that can be taken up through the digestive tract (high bioavalibility) and that can easily be assimilated. It is interesting to note that these processes may be optimized by providing nutrients in a specific, bioavailable form and/or by providing nutrients that have mutual and/or reciprocal effects with respect to bio-availability and assimilation.

According to an embodiment, the nutritional composition of the present invention comprises DHA. Preferably, the nutritional composition comprises at least 10 mg (milligram) DHA per 100 g of the dry weight of the nutritional composition. According to an embodiment, the nutritional composition comprises 10-40, more preferably 15-30, and most preferably 20-25 mg DHA per 100 g of the dry weight of the nutritional composition.

"Dry weight", for the purpose of the present specification, refers to the dry matter of the nutritional composition from which all water has been removed.

Expressed per 100 kcal of nutritional formula, the nutritional composition comprises at least about 2 mg DHA per 100 kcal of the nutritional composition. According to an embodiment, the nutritional composition comprises 3-7, preferably 3.5-6.5, even more preferably 4-6, and most preferably 4.5-5.5 mg DHA per 100 kcal of the nutritional composition.

According to an embodiment, DHA is provided in the form of phospholipids rich in DHA. Phospholipids rich in DHA may be obtained from egg yolk. In this type of DHA, DHA is bound to and thereby part of the phospholipid. The DHA is mainly esterified to phosphatidylcholine (PC) and/or phosphatidylethanolamine (PE). Phospholipid-bound DHA has the same structure as found in human brain.

Without wishing to be bound by theory, it is believed that phospholipids rich in DHA are a suitable way of providing highly bioavailable DHA to children of 1 year and older. It is believed that this form of DHA, in presence of other nutrients present in the nutritional composition of the present invention, contributes to the beneficial effects referred to in this specification.

According to an embodiment, the nutritional composition of the invention comprises an ingredient, which comprises phospholipids rich in DHA. Preferably, the ingredient comprises egg yolk lecithin containing DHA. Preferably, the ingredient comprises 80-100%, more preferably 85-95% of phospholipids by weight of total lipids.

Preferably, the ingredient comprises 50 to 90%, preferably 60 to 85%, more preferably 65-80% phosphatidylcholine by weight of the ingredient. For example, in this ingredient up to about 95%, for example up to 97% by weight of phospholipids may be provided in the form of phosphatidylcho line and phosphatidylethanolamine.

The ingredient comprising mainly phospholipids rich in DHA, may contain, for example, about 5 to about 30%, preferably 10 to 20%, more preferably 11-15% DHA by weight of total fatty acids in the ingredient. The specific ingredient may thus comprise substantial amounts of other fatty acids, such as, for example palmitic acid, oleic acid, stearic acid, linoileic acid. For example, each of said other fatty acids may be present in amounts of more than 10% by weight of total fatty acids in the said ingredient.

The nutritional composition preferably comprises ARA. ARA may be added independently from DHA and at quantities independent from the quantity of DHA added. According to an embodiment, however, the nutritional composition comprises about the same amounts of DHA and ARA. For example, the weight ratio of DHA:ARA is in the range of 2:1 to 1:2, preferably 1.5:1 to 1:1.5.

Preferably, the nutritional composition comprises at least 10 mg (milligram) ARA per 100 g of the dry weight of the nutritional composition. According to an embodiment, the nutritional composition comprises 10-45, more preferably 15-30, and most preferably 20-25 mg ARA per 100 g of the dry weight of the nutritional composition.

The nutritional composition of the present invention preferably comprises linoleic acid (LA: 18:2 ω-6), α-linolenic acid (ALA:18:3 ω-3) or both. LA and ALA are both essential long chain polyunsaturated fatty acids (LC-PUFAs). As mentioned above, these fatty acids may be transformed to DHA by the body.

Accordingly, the nutritional composition preferably comprises at least 1 g linoleic acid per 100 g of the dry weight of the nutritional composition. Preferably, the nutritional composition comprises from 2-8 g, more preferably 3-7, and most preferably 3.5-5 g of linoleic acid per 100 g of the dry weight of the nutritional composition.

According to an embodiment, the nutritional composition preferably comprises at least 100 mg α-linolenic acid per 100 g of the dry weight of the nutritional composition. Preferably, the nutritional composition comprises 200-700 mg, more preferably 300-600 mg, and most preferably 400-550 mg of α-linolenic acid per 100 g of the dry weight of the nutritional composition.

Preferably, the ratio of linoleic acid to α-linolenic acid in the nutritional composition of the invention is in the range (including endpoints) of 5:1 to 12:1, preferably 7:1 to 11:1, more preferably 8:1 to 10:1.

According to an embodiment, the ratio of ω-6 to ω-3 fatty acids in the nutritional composition of the present invention is 3:1 and 12:1, more preferably between 4:1 and 11:1, even more preferably 5:1 and 10:1. Preferably the ratio of ω-6 to ω-3 fatty acids is at least 4.5, more preferably at least 5. The human body cannot convert fatty acids from ω-6 to ω-3 and *vice versa.* Furthermore, linoleic acid and α-linolenic acid compete for the same enzymes of the metabolism, and they both inhibit each other's desaturation and elongation. Therefore, it is important to provide a balanced ratio of these essential fatty acids in the diet to ensure optimal growth and development.

According to an embodiment, the nutritional composition comprises sphingomyelin. Sphingomyelins are a subgroup of sphingophospholipids, which in turn are part of the larger group of sphingolipids in general. Sphingomyelins consist of a ceramide moiety (for example sphingosine) and a phosphacholine as a polar head group. Sphingomyelin is a structural component of cellular membranes and is found in the membranes of neurons and glia cells, and also in axons. Sphingomyelin facilitates the efficient and rapid propagation of nerve electrical signals. In particular, sphingomyelin is a component of the myelin sheath surrounding the nerve cell axons. In the present nutritional composition, sphingomyelin is mainly naturally provided as a natural component of milk, in particular cow's milk, the latter being preferably used for the preparation of the composition.

The term "naturally delivered" or "naturally provided", for the purpose of the present specification, indicates that a respective nutrient is comprised in the nutritional composition by way of the ingredients that are used to prepare the nutritional composition. It generally indicates that a respective nutrient is not specifically added in an isolated or highly concentrated form.

The nutritional composition of the invention preferably comprises at least 20 mg sphingomyelin per 100 g of the dry weight of the nutritional composition. According to an embodiment, the nutritional composition comprises 20-200, preferably 25-150, more preferably 30-100 mg sphingomyelin per 100 g of the dry weight of the nutritional composition.

According to an embodiment, the nutritional composition comprises sialic acid. Sialic acids are a family of over 50 members of 9-carbon sugars, all of which are derivatives of neuraminic acid. The predominant sialic acid family found in humans is from the N-acetylneuraminic acid sub-family. Sialic acids are contained in milk, such as cow's and goat's milk, for example. In mammals, neuronal cell membranes have the highest concentration of sialic acid compared to the other body cell membranes. The majority of sialic acid in the brain is bound to gangliosides (65%), followed by glycoproteins (32%), with less than 3% as free form. Gangliosides are cell membrane components consisting of a lipid portion and a carbohydrate portion containing at least one or more sialic acid residues. Sialic acid typically occupies the terminal position of the oligosaccharide chain serving as the first point of contact of approaching cells. Sialic acid plays an essential role in the cell to cell interaction, communication, transmission and storage of information in the brain. In the present nutritional composition, sialic acid may be added separately, but is preferably provided in sufficient amounts by way of milk components used as ingredients of the nutritional composition (naturally provided). Preferably, the nutritional composition comprises at least 50 mg of sialic acid per 100 g of dry weight of the nutritional composition. According to an embodiment, the nutritional composition comprises 60-200, preferably 70-150, more preferably 80-110 mg sialic acid per 100 g of the dry weight of the nutritional composition.

According to an embodiment, the nutritional composition comprises choline. Choline is a vitamin-like compound that is an essential part of the human diet as it is used by the body to produce acetylcholine. The human brain is rich in choline containing compounds. Choline is present in gray matter (10% of dry weight), white matter (8% of dry weight) and is a component of myelin (12% of dry weight). Without choline, acetylcholine cannot be produced. Acetylcholine is one of the crucial brain chemicals involved in memory. The most abundant brain membrane phosphatide, phsophatidylcholine, requires three circulating precursors: choline; a pyrimidine (e.g. uridine); and a polyunsaturated fatty acid. Thus choline is an extremely important structural element for cells, especially cell membranes, and is essential for the process of breaking down fat for energy. In the nutritional composition of the invention, choline is preferably not specifically added but is contained and therefore naturally provided by other ingredients, such as milk components and/or from ingredient comprising phospholipids rich in DHA mentioned above, for example.

The nutritional composition of the invention preferably comprises at least 20 mg of choline per 100 g of the dry weight of the nutritional composition. According to an embodiment, the nutritional composition comprises 20-90, preferably 30-70, more preferably 35-60 mg choline per 100 g of the dry weight of the nutritional composition.

According to an embodiment, the nutritional composition comprises thiamine. Thiamin, also known as vitamin B₁, is a water soluble vitamin of the B vitamin family. Thiamin functions as part of a coenzyme in energy-yielding systems, especially those involved in the metabolism of carbohydrates and to the breakdown of glucose to energy. Thiamin also plays a role in the conduction of nerve impulses. Studies showed that deficiencies in thiamine may lead to decrease of short term memory, confusion and irritability (behavioural problems), besides negative effects on muscles and the cardiovascular system. The composition preferably comprises at least 0.2 mg thiamin per 100 g of dry weight of the nutritional composition. According to an embodiment, the composition comprises 0.4-1.5, preferably 0.6-1.3, more preferably 0.8-1.1 mg thiamin per 100 g of dry weight of the nutritional composition.

According to an embodiment, the nutritional composition comprises folic acid. Folic acid, also known as vitamin B₉, is required for cell division and maintenance, formation of DNA and RNA, and the synthesis and breakdown of amino acids. Folic acid acts as a coenzyme in the transfer of one-carbon units. In infants and children, folate deficiency can slow overall growth. It is assumed, for the purpose of the present specification, that folate up-take is associated with cognitive performance, such as memory performance. The composition preferably comprises at least 50µg folate per 100 g of dry weight of the nutritional composition. According to an embodiment, the composition comprises 60-200, preferably 90-190, more preferably 100-170, most preferably 120-160 µg thiamin per 100 g of dry weight of the nutritional composition.

According to an embodiment, the nutritional composition comprises vitamin D. There is biological evidence to suggest an important role for vitamin D in brain development and function. The composition preferably comprises at least 1.5 µg vitamin D per 100 g of dry weight of the nutritional composition. According to an embodiment, the composition comprises 2-10, preferably 3.5-9, more preferably 5-8 µg vitamin D per 100 g of dry weight of the nutritional composition.

According to an embodiment, the nutritional composition comprises iron. Generally, iron is an essential component of several cellular functions such as the transport of oxygen, mitochondrial electron transport and DNA synthesis. Brain tissue is overall rich in iron with concentrations differing according to brain structure and stage of development. Iron is also involved in the production of myelin, in the synthesis of neurotransmitters and their breakdown. For example, iron is essential for the synthesis of dopamine, an important neurotransmitter for attention, executive functioning and motivation. Iron has been implicated in the organization of axonal growth and synaptogenesis. Today, iron deficiency is the most common single nutrient deficiency in the world. Associations between iron deficiency anaemia and deficit in cognitive or behavioural performance in children have been observed, such as lower mood and an inability to concentrate and remember as well as poor motor development. Iron is thus important for brain development.

Iron, if added as a nutritional supplement to a nutritional composition, is not always easily absorbed in the digestive tract. There are forms of iron that have low bioavailability. Therefore, according to an embodiment, iron is added in a form having high bioavailability. Iron may be provided in the form of ferric or ferrous iron (III+ and II+, respectively). AN example for added ferric iron is Iron Pyrophosphate. Preferably, iron is provided in the form of a salt comprising ferrous iron (iron with the oxidation state II+). For example, iron is provided in the form of added ferrous sulphate, including hydrates of ferrous sulfate.

Iron may also be added as iron-enriched yeast. Such products are commercially available from the companies Lesaffre and Lallemand and from the Japanese company Oriental Yeast Co. Ltd. (http://www.oyc.co.jp/e/fre 0401.htm).

According to an embodiment, the nutritional composition of the present invention comprises at least 2 mg iron per 100 g of dry weight of the nutritional composition. According to an embodiment, the composition comprises 2-10, preferably 3-8, more preferably 5-7 mg of iron per 100 g of dry weight of the nutritional composition.

According to an embodiment, the nutritional composition comprises iodine. Iodine is a trace element that is necessary for normal production of thyroid hormones, normal growth and normal brain development. Studies show that iodine deficiency may lead to mental impairment such as manifested by poor school performance reduced intellectual ability and impaired work capacity. In certain geographical areas, soil and water is naturally low in iodine, raising the risk of deficiencies in the population living in these areas. According to an embodiment, the present nutritional composition comprises at least 40 µg (microgram) of iodine per 100 g of dry weight of the nutritional composition. According to an embodiment, the composition comprises 40-150, preferably 60-120, more preferably 80-110, and most preferably 90-100 µg of iodine per 100 g of dry weight of the nutritional composition.

According to an embodiment, the nutritional composition of the present invention comprises zinc. Zinc (Zn) is an essential trace mineral that needs to be provided by the diet. Zinc is required for the biological function of more than 300 enzymes and stabilizes the tertiary structure of some proteins. In the central nervous system, zinc is concentrated in the synaptic vesicles of specific glutaminergic neurons, which are found primarily in the forebrain and connect with other cerebral structures. Furthermore, in the synapse, zinc appears to be involved in neurotransmission.

According to an embodiment, the nutritional composition of the present invention comprises at least 2 mg of zinc per 100 g of dry weight of the nutritional composition. According to an embodiment, the composition comprises 2-10, preferably 3.5-9, more preferably 5-8 mg of zinc per 100 g of dry weight of the nutritional composition.

According to an embodiment, the nutritional composition comprises at least one probiotic. Probiotics are living microorganisms which, when administered in adequate amounts confer a health benefit on the host. According to an embodiment, the nutritional composition comprises at least two different probiotic microorganisms. Preferably, each of said microorganisms is provided at about 1x10⁵ to 1x10¹² CFU per g of dry matter of the composition, preferably at least 5x10⁵ to 1x10⁹, more preferably at least 1x10⁶. The "x" means "times" and thus refers to a multiplication of the respective numbers. Preferably, a combination of two or more different probiotic microorganisms is selected that provides a health benefit, in particular when administered to children of one year and older, in particular children of the age classes as defined for the nutritional composition herein. Preferably, the two different probiotic microorganisms are selected from different species, more preferably from of different genus. According to an embodiment, the nutritional composition comprises the two probiotic microorganisms, one of which of the genus *Bifidobacterium* and the other of the genus *Lactobacillus.* Preferably, the composition comprises at least one or both selected from *Bifidobacterium longum* and *Bifidobacterium lactis* together with a *Lactobacillus rhamnosus* strain, for example. According to a preferred embodiment, the nutritional composition comprises the *Bifidobacterium longum* deposited as ATCC BAA-999, and the *Lactobacillus rhamnosus* strain deposited as ATCC 53103.

According to an embodiment, the nutritional composition comprises dietary fiber. Preferably, the composition comprises soluble fiber. According to an embodiment, the composition of the present invention comprises at least one prebiotic. Preferably, the nutritional comprises one or more selected from inulin, fructooligosaccharides (FOS), or a mixture of inulin and fructooligosaccharides. Preferably, the nutritional composition comprises at least 1, more preferably at least 2 and even more preferably at least 3 g soluble fiber per 100 g of dry weight of the nutritional composition. According to an embodiment, the nutritional composition comprises 1 to 10 g, preferably 2 to 7 g, more preferably 3 to 4 g of soluble fiber. Preferably, the soluble fiber is a mixture of inulin and FOS, said mixture comprising at least 50%, more preferably at least 60% by weight of dry matter of FOS.

According to a preferred embodiment, the composition of the present invention comprises one, a combination of several or all nutrients selected from the group of DHA, LA, ALA. Preferably, the composition also comprises ARA.

According to another embodiment, the composition further comprises one, a combination of several or all nutrients selected from the group of iron, zinc, and iodine.

According to an embodiment, the composition of the invention still further comprises one, a combination of several or all nutrients selected from the group of sphingomyelin, sialic acid, and choline.

According to an embodiment, the composition of the invention further comprises one, a combination of several or all nutrients selected from the group of folic acid (vitamin B₉), thiamine (vitamin B₁), and vitamin D.

In the combinations of preferred nutrients indicated above, quantities are preferably for each ingredient individually as provided within this specification.

Without wishing to be bound by theory, it is believed that key nutrients as specified herein synergistically interact if present in the combinations as specified herein and thereby achieve the advantages and benefits on mental performance as disclosed in this specification.

According to an embodiment, the nutritional composition of the invention comprises macronutrients and soluble fiber in the quantities indicated in Table 1 below.

It is noted that in the tables and claims herein, quantities are indicated as percent per 100 g of complete dry matter, from which residual water has been removed (or is not considered).

**Table 1: Preferred amounts of macronutrients and soluble fiber in the nutritional composition of the invention**

| **Nutrient** | | Unity | Weight per 100g |
|---|---|---|---|
| **Fat** | | g | 14-22 |
| | LA | g | 2-8 |
| | ALA | mg | 200-700 |
| | DHA | mg | 10-40 |
| | ARA | mg | 10-45 |
| **Protein** | | g | 12-20 |
| **Available carbohydrates** | | g | 50-64 |
| **Soluble fiber** | | g | at least 1 |

According to a preferred embodiment, the nutritional composition of the invention comprises macronutrients and soluble fiber in the quantities (percent per 100 g dry matter) indicated in Table 2 below:

**Table 2: More preferred amounts of macronutrients and soluble fiber in the nutritional composition of the invention**

| **Nutrient** | | Unity | Weight per 100g |
|---|---|---|---|
| **Fat** | | g | 15-21 |
| | LA | g | 3-7 |
| | ALA | mg | 300-600 |
| | DHA | mg | 15-30 |
| | ARA | mg | 15-30 |
| **Protein** | | g | 13-19 |
| **Available carbohydrates** | | g | 52-62 |
| **Soluble fiber** | | g | at least 2 |

According to a most preferred embodiment, the nutritional composition of the invention comprises macronutrients and soluble fiber in the quantities (percent per 100 g dry matter) indicated in Table 3 below:

**Table 3: Most preferred amounts of macronutrients and soluble fiber in the nutritional composition of the invention**

| **Nutrient** | | Unity | Weight per 100g |
|---|---|---|---|
| **Fat** | | g | 16.3-19.3 |
| | LA | g | 3.5-5 |
| | ALA | mg | 400-550 |
| | DHA | mg | 20-25 |
| | ARA | mg | 20-25 |
| **Protein** | | g | 14.5-17.5 |
| **Available carbohydrates** | | g | 54-59 |
| **Soluble fiber** | | g | at least 3 |

With respect to the micronutrients, in particular vitamins and minerals, the preferred, more preferred and most preferred quantities of key micro-nutrients for the purpose of the present invention are set out in Tables 4-9 below.

**Table 4: Preferred amounts of some key micronutrients in the nutritional composition of the invention**

| **Minerals and vitamins** | | Unity | Weight per 100g |
|---|---|---|---|
| | sphingomyelin | mg | 20-200 |
| | sialic acid | mg | 60-200 |
| | choline | mg | 20-110 |
| | thiamin | mg | 0.4-1.5 |
| | folic acid | µg | 60-200 |
| | vitamin D | µg | 2-10 |

**Table 5: More preferred amounts of some key micronutrients in the nutritional composition of the invention**

| **Minerals and vitamins** | | Unity | Weight per 100g |
|---|---|---|---|
| | sphingomyelin | mg | 25-150 |
| | sialic acid | mg | 70-150 |
| | choline | mg | 30-100 |
| | thiamin | mg | 0.6-1.3 |
| | folic acid | µg | 90-190 |
| | vitamin D | µg | 3.5-9 |

**Table 6: Most preferred amounts of some key micronutrients in the nutritional composition of the invention**

| **Minerals and vitamins** | | Unity | Weight per 100g |
|---|---|---|---|
| | sphingomyelin | mg | 30-100 |
| | sialic acid | mg | 80-110 |
| | choline | mg | 35-80 |
| | thiamin | mg | 0.8-1.1 |
| | folic acid | µg | 100-170 |
| | vitamin D | µg | 5-8 |

According to an embodiment, the nutritional compositions as defined by Tables 1-6 and combinations thereof further comprise one, two or all three selected from iron, iodine and zinc, preferably in the amounts indicated above, or in Tables 7-9 below.

**Table 7: Preferred amounts of some key micronutrients in the nutritional composition of the invention**

| **Minerals and vitamins** | | Unity | Weight per 100g |
|---|---|---|---|
| | Iron | mg | 2-10 |
| | Iodine | µg | 40-150 |
| | Zinc | mg | 2-10 |

**Table 8: More preferred amounts of some key micronutrients in the nutritional composition of the invention**

| **Minerals and vitamins** | | Unity | Weight per 100g |
|---|---|---|---|
| | Iron | mg | 3-8 |
| | Iodine | µg | 60-120 |
| | Zinc | mg | 3.5-9 |

**Table 9: Most preferred amounts of some key micronutrients in the nutritional composition of the invention**

| **Minerals and vitamins** | | Unity | Weight per 100g |
|---|---|---|---|
| | Iron | mg | 4-7 |
| | Iodine | µg | 80-110 |
| | Zinc | mg | 5-8 |

The Tables 1-9 concerning macro- and micronutrients, respectively may be combined in any way and in particular as detailed in Table 10 below in accordance with the nutritional composition of the present invention.

Table 10 below discloses various embodiments encompassed by the present invention.

**Table 10 Macro- and micronutrients according to embodiments of the present invention**

| Embodiment of the invention | Tables in the combination of Tables 1-9 |
|---|---|
| 1 | 1+4+7 |
| 2 | 1+5+7 |
| 3 | 1+6+7 |
| 4 | 1+4+8 |
| 5 | 1+5+8 |
| 6 | 1+6+8 |
| 7 | 1+4+9 |
| 8 | 1+5+9 |
| 9 | 1+6+9 |
| 10 | 2+4+7 |
| 11 | 2+5+7 |
| 12 | 2+6+7 |
| 13 | 2+4+8 |
| 14 | 2+5+8 |
| 15 | 2+6+8 |
| 16 | 2+4+9 |
| 17 | 2+5+9 |
| 18 | 2+6+9 |
| 19 | 3+4+7 |
| 20 | 3+5+7 |
| 21 | 3+6+7 |
| 22 | 3+4+8 |
| 23 | 3+5+8 |
| 24 | 3+6+8 |
| 25 | 3+4+9 |
| 26 | 3+5+9 |
| 27 | 3+6+9 |

In any event, the nutritional compositions of the invention as described in the tables above preferably comprise probiotics and, optionally, further nutrients as defined further above.

The nutritional composition preferably provides 400-500 kcal, more preferably 420-480 kcal per 100 g of dry weight of the composition. According to an embodiment, per dl of the reconstituted formula, the composition provides preferably 40-80, more preferably 45-75, most preferably 50-70 kcal. The caloric content of the nutritional composition per volume (for example 100 ml) may generally also be determined on the basis of the caloric content per 100 g and considering reconstitution quantities as indicated below.

According to an embodiment, the nutritional composition of the present invention comprises, per 100 g dry matter, the nutrients as defined in Table 11 below:

**Table 11: Embodiment of a nutritional composition of the present invention**

| **Nutrient** | | Unity | Weight per 100g of dry matter | |
|---|---|---|---|---|
| | | | Low limit value | High limit value |
| **Fat** | | g | **15.44** | **21.79** |
| | Milk fat | g | 2.95 | 4.16 |
| | Non-milk Fat | g | 11.80 | 16.66 |
| | Lecithin | g | 0.26 | 0.37 |
| | Egg phospholipids | g | 0.23 | 0.32 |
| Fatty acids provided by the above fat nutrients: | | | | |
| Linoleic acid (LA) | | g | 3.73 | 5.27 |
| α-Linolenic acid (ALA) | | mg | 408.01 | 576.01 |
| Arachidonic acid (ARA) | | mg | 16.10 | 40.00 |
| DHA | | mg | 14.70 | 29.40 |
| **Protein** | | g | **13.74** | **19.40** |
| | Casein | g | 10.58 | 14.94 |
| | Whey protein | g | 3.16 | 4.46 |
| **Available carbohydrates** | | g | **49.44** | **69.79** |
| | Lactose | g | 21.25 | 30.00 |
| | Other sugars (mainly fructose, glucose and saccharose) | g | 0.60 | 0.85 5 |
| | Maltodextrin | g | 27.58 | 38.94 |
| **Dietary fiber** | | g | **3.03** | **4.28** |
| | Inulin & oligosaccharides | g | 3.03 | 4.28 |
| **Minerals (ash)** | | g | **3.30** | **4.66** |
| Specific minerals contained in the ash: | | | | |
| | Sodium | mg | 204.00 | 288.00 |
| | Potassium | mg | 654.52 | 924.02 |
| | Chloride | mg | 471.76 | 666.01 |
| | Calcium | mg | 531.26 | 750.01 |
| | Phosphorus | mg | 412.26 | 582.01 |
| | Magnesium | mg | 46.75 | 66.00 |
| | Manganese | µg | 25.50 | 36.00 |
| | Selenium | µg | 9.35 | 13.20 |
| **Micronutrients (other)¹** | | mg | **293.30** | **414.07** |
| | Sphingomyelin² | mg | 35.00 | 70.00 |
| | Choline² | mg | 50.40 | 100.80 |
| | Sialic acid² | mg | 66.50 | 133.00 |
| | Taurine | mg | 30.60 | 43.20 |
| | Iodine³ | µg | 76.00 | 123.50 |
| | Iron | mg | 4.32 | 7.02 |
| | Zinc | mg | 5.44 | 8.84 |
| | Thiamin (B₁) | mg | 0.76 | 1.24 |
| | Niacin (vit B₃) | mg | 10.20 | 14.40 |
| | Pyridoxin (vit B₆) | mg | 1.19 | 1.68 |
| | Biotin(B₈) | µg | 22.95 | 32.40 |
| | Folic acid (vit B₉) | µg | 112.00 | 182.00 |
| | Cobalamin (vitB₁₂)² | µg | 0.77 | 1.08 |
| | Vitamin C | mg | 45.90 | 64.80 |
| | Vitamin A (retinol) | µg RE⁴ | 459.00 | 648.00 |
| | Vitamin D | µg | 5.78 | 8.16 |
| | Vitamin E | mg | 4.59 | 6.48 |
| | Vitamin K | µg | 17.00 | 24.00 |
| | Vitamin B₂ | mg | 0.94 | 1.32 |
| | Pantothenic acid | mg | 3.49 | 4.92 |
| Total solids (dry matter) | | g | **100** | |

| | | | | |
|---|---|---|---|---|
| 1) These micronutrients are also part of the ash 2) Naturally provided, in particular contained in milk ingredients 3) Partially naturally provided, other part added separately 4) RE=Retinol Equivalents | | | | |

According to a preferred embodiment, the nutritional composition of the present invention comprises, per 100 g dry matter, the nutrients as defined in Table 12 below:

**Table 12: Preferred embodiment of a nutritional composition of the present invention**

| **Nutrient** | | Unity | Weight per 100g of dry matter | |
|---|---|---|---|---|
| | | | Upper limit | Lower limit |
| **Fat** | | g | **16.34** | **20.88** |
| | Milk fat | g | 3.12 | 3.99 |
| | Non-milk Fat | g | 12.49 | 15.96 |
| | Lecithin | g | 0.28 | 0.36 |
| | Egg phospholipids | g | 0.24 | 0.31 |
| Fatty acids provided by the above fat nutrients: | | | | |
| Linoleic acid (LA) | | g | 3.95 | 5.05 |
| α-Linolenic acid (ALA) | | mg | 432.01 | 552.01 |
| Arachidonic acid (ARA) | | mg | 18.40 | 29.90 |
| DHA | | mg | 16.80 | 27.30 |
| **Protein** | | g | **14.55** | **18.60** |
| | Casein | g | 11.21 | 14.32 |
| | Whey protein | g | 3.35 | 4.28 |
| **Available carbohydrates** | | g | **52.34** | **66.88** |
| | Lactose | g | 22.50 | 28.75 |
| | Other sugars (mainly fructose, glucose and saccharose) | g | 0.64 | 0.82 |
| | Maltodextrin | g | 29.21 | 37.32 |
| **Dietary fiber** | | g | **3.21** | **4.11** |
| | Inulin & oligosaccharides | g | 3.21 | 4.11 |
| **Minerals (ash)** | | g | **3.49** | **4.46** |
| Specific minerals contained in the ash: | | | | |
| | Sodium | mg | 216.00 | 276.00 |
| | Potassium | mg | 693.02 | 885.52 |
| | Chloride | mg | 499.51 | 638.26 |
| | Calcium | mg | 562.51 | 718.76 |
| | Phosphorus | mg | 436.51 | 557.76 |
| | Magnesium | mg | 49.50 | 63.25 |
| | Manganese | µg | 27.00 | 34.50 |
| | Selenium | µg | 9.90 | 12.65 |
| **Micronutrients (other)¹** | | mg | **310.55** | **396.82** |
| | Sphingomyelin² | mg | 40.00 | 65.00 |
| | Choline² | mg | 57.60 | 93.60 |
| | Sialic acid² | mg | 76.00 | 123.50 |
| | Taurine | mg | 32.40 | 41.40 |
| | Iodine³ | µg | 80.75 | 114.00 |
| | Iron | mg | 4.59 | 6.48 |
| | Zinc | mg | 5.78 | 8.16 |
| | Thiamin (B₁) | mg | 0.81 | 1.14 |
| | Niacin (vit B₃) | mg | 10.80 | 13.80 |
| | Pyridoxin (vit B₆) | mg | 1.26 | 1.61 |
| | Biotin(B₈) | µg | 24.30 | 31.05 |
| | Folic acid (vit B₉) | µg | 119.00 | 168.00 |
| | Cobalamin (vit B₁₂)¹ | µg | 0.81 | 1.04 |
| | Vitamin C | mg | 48.60 | 62.10 |
| | Vitamin A (retinol)⁴ | µg RE² | 486.00 | 621.00 |
| | Vitamin D | µg | 6.12 | 7.82 |
| | Vitamin E | mg | 4.86 | 6.21 |
| | Vitamin K | µg | 18.00 | 23.00 |
| | Vitamin B₂ | mg | 0.99 | 1.27 |
| | Pantothenic acid | mg | 3.69 | 4.72 |
| Total solids (dry matter) | | g | **100** | |

According to a preferred embodiment, the nutritional composition of the present invention comprises, per 100 g dry matter, the nutrients as defined in Table 13 below:

**Table 13: Preferred embodiment of a nutritional composition of the present invention**

| **Nutrient** | | Unity | Weight per 100g of dry matter | |
|---|---|---|---|---|
| | | | Upper limit | Lower limit |
| **Fat** | | g | **17.25** | **19.98** |
| | Milk fat | g | 3.30 | 3.82 |
| | Non-milk Fat | g | 13.19 | 15.27 |
| | Lecithin | g | 0.29 | 0.34 |
| | Egg phospholipids | g | 0.26 | 0.30 |
| Fatty acids provided by the above fat nutrients: | | | | |
| Linoleic acid (LA) | | g | 4.17 | 4.83 |
| α-Linolenic acid (ALA) | | mg | 456.01 | 528.01 |
| Arachidonic acid (ARA) | | mg | 19.55 | 25.30 |
| DHA | | mg | 17.85 | 25.20 |
| **Protein** | | g | **15.36** | **17.79** |
| | Casein | g | 11.83 | 13.70 |
| | Whey protein | g | 3.53 | 4.09 |
| Available carbohydrates | | g | **55.25** | **63.98** |
| | Lactose | g | 23.75 | 27.50 |
| | Other sugars (mainly fructose, glucose and saccharose) | g | 0.67 | 0.78 |
| | Maltodextrin | g | 30.83 | 35.70 |
| **Dietary fiber** | | g | **3.39** | **3.93** |
| | Inulin & oligosaccharides | g | 3.39 | 3.93 |
| **Minerals (ash)** | | g | **3.69** | **4.27** |
| Specific minerals contained in the ash: | | | | |
| | Sodium | mg | 228.00 | 264.00 |
| | Potassium | mg | 731.52 | 847.02 |
| | Chloride | mg | 527.26 | 610.51 |
| | Calcium | mg | 593.76 | 687.51 |
| | Phosphorus | mg | 460.76 | 533.51 |
| | Magnesium | mg | 52.25 | 60.50 |
| | Manganese | µg | 28.50 | 33.00 |
| | Selenium | µg | 10.45 | 12.10 |
| **Micronutrients (other)¹** | | mg | **327.81** | **379.57** |
| | Sphingomyelin² | mg | 42.50 | 60.00 |
| | Choline² | mg | 61.20 | 86.40 |
| | Sialic acid² | mg | 80.75 | 114.00 |
| | Taurine | mg | 34.20 | 39.60 |
| | Iodine³ | µg | 85.50 | 109.25 |
| | Iron | mg | 4.86 | 6.21 |
| | Zinc | mg | 6.12 | 7.82 |
| | Thiamin (B₁) | mg | 0.86 | 1.09 |
| | Niacin (vit B₃) | mg | 11.40 | 13.20 |
| | Pyridoxin (vit B₆) | mg | 1.33 | 1.54 |
| | Biotin(B₈) | µg | 25.65 | 29.70 |
| | Folic acid (vit B₉) | µg | 126.00 | 161.00 |
| | Cobalamin (vit B₁₂)¹ | µg | 0.86 | 0.99 |
| | Vitamin C | mg | 51.30 | 59.40 |
| | Vitamin A (retinol)⁴ | µg RE² | 513.00 | 594.00 |
| | Vitamin D | µg | 6.46 | 7.48 |
| | Vitamin E | mg | 5.13 | 5.94 |
| | Vitamin K | µg | 19.00 | 22.00 |
| | Vitamin B₂ | mg | 1.05 | 1.21 |
| | Pantothenic acid | mg | 3.90 | 4.51 |
| Total solids (dry matter) | | g | **100** | |

In Tables 11-13, the indications 1)-4) have the same meaning (see bottom of Table 11).

According to an embodiment, the nutritional composition of the present invention is adapted to children of an age of 3 years or older.

Preferably, the nutrients provided in the nutritional composition are adapted to the needs and nutritional recommendations of children of a specific age. For children that have an age of 3 years or older, for example 3-6, preferably 3-5 and most preferably 3-4 years, the amounts of some nutrients in the nutritional composition are preferably adapted, while other nutrients are the same as discussed above. The amounts of nutrients that are preferably adapted in view of the beneficial properties and advantages obtained with respect to the mental performance of a child are specified below, whereas the contents and amounts of those nutrients that are not further discussed below (for example, sialic acid) can be taken from the amounts disclosed above.

With respect to the macronutrients, it is noted that children of 3 years and older generally have higher requirements in proteinogenic matter (protein source) and lower requirements in carbohydrates and slightly higher requirements in than children of more than 1 and up to three years. According to an embodiment, said protein source provides about 13 to about 23, preferably 16-20 percent of the total energy, said source of available carbohydrates provides from about 33 to about 50, preferably 40-46 percent of the total energy, and said lipid source provides from about 38 to about 48, preferably 37-44 percent of the total energy of the composition.

In terms of parts of dry matter, the composition comprises preferably 16-26 g, more preferably 17-24 g, and most preferably 18-22 g fats per 100 g of dry matter of the composition.

The composition preferably comprises 39-55 g, more preferably 41-53 g, and most preferably 44-50 g available carbohydrates per 100 g of dry matter of the composition.

The composition preferably comprises 16-25 g, more preferably 17-24 g, and most preferably 18-22 g of proteinogenic mater (protein source) per 100 g of dry matter of the composition.

With respect to DHA, the nutritional composition for children of 3 or more years as defined above comprises at least ≥ 0 mg DHA per 100 kcal of the nutritional composition. According to an embodiment, the nutritional composition comprises 0.5-20, preferably 0.5-10, even more preferably 0.5-8 mg DHA per 100 kcal of the nutritional composition.

With respect to sphingomyelin the nutritional composition for children of 3 or more years as defined above comprises at least 10mg sphingomyelin per 100 g of the dry weight of the nutritional composition. According to an embodiment, the nutritional composition comprises 15-250, preferably 20-150, more preferably 20-90 mg sphingomyelin per 100 g of the dry weight of the nutritional composition.

With respect to choline, the nutritional composition for children of 3 or more years as defined above comprises at least 10mg choline per 100 g of the dry weight of the nutritional composition. According to an embodiment, the nutritional composition comprises 12-100, preferably 15-80, more preferably 18-50 mg choline per 100 g of the dry weight of the nutritional composition.

With respect to iron, the nutritional composition for children of 3 or more years as defined above comprises at least 2.5 mg iron per 100 g of dry weight of the nutritional composition. According to an embodiment, the composition comprises 2.5-12, preferably 3.5-10, more preferably 5-8.5 mg of iron per 100 g of dry weight of the nutritional composition.

With respect to iodine, the nutritional composition for children of 3 or more years as defined above comprises at least 60 µg (microgram) of iodine per 100 g of dry weight of the nutritional composition. According to an embodiment, the composition comprises 60-160, preferably 70-140, more preferably 90-130 µg of iodine per 100 g of dry weight of the nutritional composition.

With respect to zinc, the nutritional composition for children of 3 or more years as defined above comprises at least 5 mg of zinc per 100 g of dry weight of the nutritional composition. According to an embodiment, the composition comprises 5-16, preferably 6-14 mg of zinc per 100 g of dry weight of the nutritional composition.

The nutritional composition of the present invention may be provided as a ready-to-drink liquid form or in the form of a dry powder to be reconstituted with water, or another water containing liquid (less preferred). Preferably, the nutritional composition is provided in powdered form, and reconstituted by addition of water by the consumer shortly (for example 1 minute to 2 hours) before consumption. As is usual, the water has to be clean, that is preferably bottled water or has been boiled and filtered and cooled to 60°C or lower before addition to the nutritional composition.

If the nutritional composition is provided in the form of a powder, it is preferably reconstituted by mixing 12-20 g powder in 1 dl water, preferably 14-20 g powder, more preferably about 16-18 g powder in 1 dl (0.1 liter) water.

The typical serving size is about 200 ml, that is, in two times the quantity defined above (for example, 34 grams) is added to 200 ml water. Per day, ideally 2 to 3 serving sizes are consumed per child of 1 to 3 years.

According to a preferred embodiment, the nutritional composition is a growing up milk, also referred to as a GUM. More preferably, the nutritional composition is a powdered GUM, preferably substantially free of starch.

The nutritional composition provides several benefits associated with the healthy mental, behavioural and intellectual development of the child.

In an aspect the nutritional composition supports and/or improves overall cognitive performance, for example cognitive capacities and/or abilities in a child.

In an aspect, the nutritional composition is used to provide nutrients that have at least one of the following purposes: nutrients that (1) the body of a child can use to structurally build up the brain, (2) are needed for the biochemical processes taking place in the brain, and/or (3) that provide the energy necessary for the functioning of the brain. Preferably, the nutritional composition provides nutrients for (1), for (1) and (2), for (1) and (3), for (2) and (3) or for (1), (2) and (3) above.

According to an aspect, the nutritional composition provides nutrients or a combination of nutrients that is bioavailable. Preferably, the nutrients have increased bioavailability if compared to nutrients provided in a different, for example conventional form, or in a different combination of nutrients. Preferably, the nutrients are provided in a form that is actually absorbed in the digestive tract and used by the body to build up the brain. In particular, DHA is provided in the form of phospholipids rich in DHA, which is considered to be highly bioavailable and which is believed to be carried to the brain.

In an aspect, the nutritional composition supports the healthy, normal or improved behavioural, cognitive, communicational, such as language, expressive and receptive communication, psychomotor and social-emotional development of a child.

In an aspect, the nutritional composition supports and/or improves cognitive development of a child. In particular, the nutritional composition supports one or more selected from sensorimotor development, exploration and manipulation, object relatedness, concept formation, memory, habituation, visual acuity, visual preference, object recognition, visual attention and/or other aspects of cognitive processing. In dependence of the age, the nutritional composition supports and/or improves cognitive development and/or capacities in one or more of the areas selected from counting (with one-to-one correspondence and cardinality), visual and tactile exploration, object assembly, puzzle board completion, matching colors, comparing masses, representational and/or pretend play, discriminating patterns.

With respect to language skills, the nutritional composition of the present invention supports and/or improves the development and/or abilities of expressive communication, preverbal communications such as babbling, gesturing, joint referencing, turn taking, vocabulary development such as naming objects, pictures, using words to make needs and/or wants known, and actions, morpho-syntactic development such as use of two-word utterances and use of plurals and verb tense, receptive communication, preverbal behaviors and vocabulary development such as the ability to identify objects and pictures that are referenced, vocabulary related to morphological development such as pronouns and prepositions, understanding of morphological markers such as plurals and tense markings.

In an aspect, the nutritional composition supports and/or improves social-emotional development and/or abilities. Depending on the specific age of an infant or a child, the nutritional composition supports and/or improves the child's self-regulation, interest in the world, engagement in relationships, use of emotions in an interactive, purposeful manner, use of interactive, emotional signals or gestures to communicate, use of interactive, emotional signals or gestures to solve problems, use of ideas to convey feelings, wishes, or intentions, the creation of logical bridges between emotions and ideas.

In an aspect, the nutritional composition supports and/or improves achievement visual skills, auditory skills and adaptive behavior.

In an aspect, the nutritional composition of the invention supports and/or improves memory, in particular short term memory, working memory and/or long term memory, preferably at least short term memory and/or working memory. Short term memory and working memory can be more readily tested in typical experimental settings.

"Memory" is an organism's mental ability to store, retain and recall information. For the purpose of the present specification, memory is one, two (for example, (a) and (c)) or all three selected from (a) to store, (b) retain and (c) recall information. Memory phenomena that can be examined for the purpose of the present specification include: (1) knowledge (what is remembered?); (2) comprehension (what does it mean?); (3) context/function (why remember?); and (4) strategy (how to remember?). Memory is complex psychological process that is not independent of a single memory domain process. Memory is related to several other cognition domains including, sensory memory, audio memory and visual memory. The terms of "memory" include:
"Short-term memory": a system for temporarily, for example up to 60 (=1 minute), up to 30, up to 20 or up to 15 seconds, storing and managing information required to carry out cognitive tasks such as learning, reasoning, and comprehension, for example.
"Working memory": refers to short-term memory and requires the simultaneous storage and processing of information.
"Long-term memory" is memory that can last beyond a few, for example beyond 2-3, minutes to a few decades. Items of information stored as long-term memory may be available for a lifetime.

Memory is generally understood to be a process by which what is learned persists across time. In this sense, learning and memory are inextricably connected. Accordingly, the composition of the present invention supports and/or improves a child's learning and/or memory capacities.

For example, the nutritional composition of the present invention supports and/or increases a child's capacity recall words, numbers, pictures, symbols, letters, and/or tonal sequences within the short-term.

According to an aspect, the nutritional composition supports and/or improves at least one selected from learning capacity, alertness, attentiveness, and concentration capacity of a child.

According to an aspect, the composition of the present invention supports and/or improves a child's skills and/or abilities in one or more of (1) the comprehension and use of language, (2) mathematical abilities, (3) the conceptualization and reasoning without words, and (4) short term memory and/or long term memory.

The benefits reported herein are generally obtained when consuming the nutritional composition of the invention repeatedly over a certain period of time, for example at least 1 week, at least one month, at least 45 days, at least 3 months most preferably at least 6 months, for example at least 9 months, even more preferably at least 1 years. The time period is preferably sufficiently large so that at least some kind of development and/or growth takes place in the consumer. Preferably, the nutritional composition is consumed repeatedly and even more preferably on a regular basis. Preferably, at least one serving size of the nutritional is consumed at least on 2, 3, 4, 5, 6, or 7 different days of a week. More preferably, 2-3 serving sizes are consumed at least on 2, 3, 4, 5, 6, or 7 different days of a week for the time periods indicated above. It is, of course, not necessary to consume the composition the same number of days every week, although daily consumption is, of course, preferred.

The above examples of cognitive, language and social-emotional skills may be assessed, for example, using statistical tests designed for infants and children, such as the Bayley Scales of Infant and Toddler Development®, Third Edition (Bayley-III®), of Nancy Bayley, Ph.D. or also using the modified Bayley II test, for example. These tests can be used to assess infants and children of 1 to 42 months of age, with subtests directed to specific age ranges, for example 2 to 2.5 years of age.

Another suitable test for assessing performance, such as cognitive and the like are the McCarthy Scales *(McCarthy Scales of Children's Abilities.* New York, NY: Psychological Corp; 1972 of Dorothea McCarthy). This test is useful to assess abilities of children of the age of 2.5 to 8.5 years.

The following examples are illustrative of some of the products and methods of making the same falling within the scope of the present invention. They are not to be considered in any way limitative of the invention. Changes and modifications can be made with respect to the invention. That is, the skilled person will recognize many variations in these examples to cover a wide area of formulas, ingredients, processing and mixtures to rationally adjust the nutrients and other elements of the invention for a variety of applications.

### Examples

### Example 1: Nutritional composition for children of 1-2 years according to the invention

A powdered milk for toddlers of 1 to 3 years is produced by preparing a slurry of ingredients, in particular skimmed milk, whole milk, maltodextrin, sugar, vegetable oil, flavouring ingredients, an emulsifier, a vitamin mix and phospholipids rich in DHA. The slurry is subjected to heating for evaporation and then transformed to a powder by spray-drying.

The final composition of the nutrients of the growing up milk per 100 grams of dry matter is provided in Table 14 below.

The composition of Table 14 contains about 450 kcal per 100 g dry matter.

Thereafter, two different probiotic microorganisms, *Bifidobacterium longum* (ATCC BAA-999) and *Lactobacillus rhamnosus* (ATCC 54193) in powdered form are added so as to each provide at least 1x10⁶ CFU per gram of nutritional composition.

**Table 14: Nutrients of the nutritional composition for children of 1-2 years**

| **Nutrient** | | Unity | Weight per 100g of dry matter |
|---|---|---|---|
| **Fat** | | g | **18.16** |
| | Milk fat | g | 3.47 |
| | Non-milk Fat | g | 13.88 |
| | Lecithin | g | 0.31 |
| | Egg phospholipids | g | 0.27 |
| Fatty acids provided by the above fat nutrients: | | | |
| Linoleic acid (LA) | | g | 4.39 |
| α-Linolenic acid (ALA) | | mg | 480.01 |
| Arachidonic acid (ARA) | | mg | 23.00 |
| DHA | | mg | 21.00 |
| **Protein** | | g | **16.17** |
| | Casein | g | 12.45 |
| | Whey protein | g | 3.72 |
| **Available carbohydrates** | | g | **58.16** |
| | Lactose | g | 25.00 |
| | Sugars (fructose, glucose and saccharose) | g | 0.71 |
| | Maltodextrin | g | 32.45 |
| **Dietary fiber** | | g | **3.57** |
| | Inulin & oligosaccharides | g | 3.57 |
| **Minerals (ash)** | | g | **3.88** |
| Specific minerals contained in the ash: | | | |
| | Sodium | mg | 240.00 |
| | Potassium | mg | 770.02 |
| | Chloride | mg | 555.01 |
| | Calcium | mg | 625.01 |
| | Phosphorus | mg | 485.01 |
| | Magnesium | mg | 55.00 |
| | Manganese | µg | 30.00 |
| | Selenium | µg | 11.00 |
| **Micronutrients (other)¹** | | mg | **345.06** |
| | Sphingomyelin² | mg | 50.00 |
| | Choline² | mg | 72.00 |
| | Sialic acid² | mg | 95.00 |
| | Taurine | mg | 36.00 |
| | Iodine³ | µg | 95.00 |
| | Iron | mg | 5.40 |
| | Zinc | mg | 6.80 |
| | Thiamin (B₁) | mg | 0.95 |
| | Niacin (vit B₃) | mg | 12.00 |
| | Pyridoxin (vit B₆) | mg | 1.40 |
| | Biotin (B₈) | µg | 27.00 |
| | Folic acid (vit B₉) | µg | 140.00 |
| | Cobalamin (vit B₁₂)¹ | µg | 0.90 |
| | Vitamin C | mg | 54.00 |
| | Vitamin A (retinol)⁴ | µg RE² | 540.00 |
| | Vitamin D | µg | 6.80 |
| | Vitamin E | mg | 5.40 |
| | Vitamin K | µg | 20.00 |
| | Vitamin B₂ | mg | 1.10 |
| | Pantothenic acid | mg | 4.10 |
| Total solids (dry matter) | | g | **100** |

| | | | |
|---|---|---|---|
| 1) These micronutrients are also part of the ash. 2) Naturally provided, in particular contained in milk ingredients. 3) Partially naturally provided, other part added separately with the vitamin mix. 4) RE=Retinol Equivalents. | | | |

### Example 2: Nutritional composition for children of 3-5 years according to the invention

A powdered milk for children of 3 to 5 years is produced by preparing a slurry of ingredients, in particular skimmed milk, whole milk, maltodextrin, sugar, vegetable oil, flavouring ingredients, an emulsifier, a vitamin mix and phospholipids rich in DHA. The slurry is subjected to heating for evaporation and then transformed to a powder by spray-drying.

The final composition of the nutrients of the growing up milk per 100 grams of dry matter is provided in Table 15 below.

The composition of Table 15 contains about 450 kcal per 100 g dry matter.

Thereafter, two different probiotic microorganisms, *Bifidobacterium longum* (ATCC BAA-999) and *Lactobacillus rhamnosus* (ATCC 54193) in powdered form are added so as to each provide at least 1x10⁶ CFU.

**Table 15: Nutrients of the nutritional composition for children of 3-5 years**

| **Nutrient** | | Unity | Weight per 100g of dry matter |
|---|---|---|---|
| **Fat** | | g | **19.80** |
| | Milk fat | g | 4.39 |
| | Non-milk Fat | g | 14.80 |
| | Lecithin | g | 0.31 |
| | Egg phospholipids | g | 0.3 |
| Fatty acids provided by the above fat nutrients: | | | |
| Linoleic acid (LA) | | g | 4.39 |
| α-Linolenic acid (ALA) | | mg | 459.18 |
| Arachidonic acid (ARA) | | mg | 43.88 |
| DHA | | mg | 29.59 |
| **Protein** | | g | **21.43** |
| | Casein | g | 16.63 |
| | Whey protein | g | 4.80 |
| **Available carbohydrates** | | g | **50.00** |
| | Lactose | g | 30.61 |
| | Saccharose | g | 0.10 |
| | Maltodextrin | g | 19.29 |
| **Dietary fiber** | | | **3.27** |
| | Inulin & oligosaccharides | g | |
| **Minerals (ash)** | | g | **5.51** |
| Specific minerals contained in the ash: | | | |
| | Sodium | mg | 331.63 |
| | Potassium | mg | 1230.61 |
| | Chloride | mg | 755.10 |
| | Calcium | mg | 1142.86 |
| | Phosphorus | mg | 653.06 |
| | Magnesium | mg | 91.84 |
| | Manganese | µg | 30.61 |
| | Selenium | µg | 11.73 |
| **Micronutrients (other)¹** | | mg | |
| | Sphingomyelin² | mg | 51.02 |
| | Choline² | mg | 73.47 |
| | Sialic acid² | mg | 96.94 |
| | Taurine | mg | 36.73 |
| | Iodine³ | µg | 96.94 |
| | Iron | mg | 5.51 |
| | Zinc | mg | 6.94 |
| | Thiamin (B₁) | mg | 0.97 |
| | Niacin (vit B₃) | mg | 12.24 |
| | Pyridoxin (vit B₆) | mg | 1.43 |
| | Biotin(B₈) | µg | 27.55 |
| | Folic acid (vit B₉) | µg | 142.86 |
| | Cobalamin (vit B₁₂₎² | µg | 0.92 |
| | Vitamin C | mg | 55.10 |
| | Vitamin A (retinol) | µg RE⁴ | 551.02 |
| | Vitamin D | µg | 6.94 |
| | Vitamin E | mg | 5.51 |
| | Vitamin K | µg | 20.41 |
| | Vitamin B₂ | mg | 1.12 |
| | Pantothenic acid | mg | 4.18 |
| Total solids (dry matter) | | g | **100** |

In Table 15, the indications 1)-4) have the previous meaning (see bottom of Table 14).

### Example 3: Clinical Studies For Assessing Mental and Cognitive Performance of Children of 1.5 to about 2.5 years of age

In a clinical study, the cognitive, language, motor, adaptive behaviour and social-emotional skills of children are to be assessed.

For the study, a randomized, controlled, double blind, multi centre design with two parallel groups is used. A total of 400 healthy children of the age of 16 to 18 months of both sexes are recruited. The toddlers are split in two groups. One group (n=200) receives the growing up milk as detailed in Example 1, the other group is the control group and receives milk powder. Both groups take up food, in particular food as usual. The children will receive the nutritional composition of the invention milk at 2 serving sizes per day. One serving size is obtained by mixing 36 g of powder with 200 ml water. The children of the control group receive an equivalent amount of the control powder. Duration of subject participation is 12 month.

Growth assessments in all children include body weight, body height and head circumference.

Cognitive function is assessed at three different time points: at the beginning (baseline, 18 months age), 6 months after the start (at the age of 2 years) and 12 months after the start of the study (2.5 years of age). At all time points, the children's healthy development in the general areas of adaptive behavior, cognition, language, motor, and social-emotional abilities and development are assessed using the established Bayley Scales of Infant and Toddler Development®, Third Edition (Bayley-III®), of Nancy Bayley, Ph.D., which is commercially available at:
(http://pearsonassess.com/HAIWEB/Cultures/en-us/Productdetail.htm?Pid=015-8027-23X&Mode=summary).

At the last timepoint (2.5 years of age) the test McCarthy Scales of Children's Abilities will be also used. The test contains four subtests for memory to test short-term recall of words, numbers, pictures, and tonal sequences.

The tests are adapted to the child's culture and language. According to the protocol of this test, each child's capacity is assessed separately.

Furthermore, biological samples are taken. Two blood samples for measuring the lipid and micronutrient profile, such as sialic acid, choline concentrations, etc. Stool samples are taken for determining stool pH, stool IgA concentration, stool bacterial profile and the presence of *Bifidobacteria, Lactobacillus* and *B. lactis* are assessed.

The health of all children is monitored throughout the study.

At the end of the study, the comparison between the groups shows that the group receiving the nutritional composition exhibit better overall development and cognitive ability and performance. Generally, the children receiving the composition of the invention perform better in the areas assessed by the test, in particular in the areas of cognitive ability and performance, language skills, psychomotor and social-emotional development.

In the area of cognitive ability and performance, it is, for example, shown that the children receiving the nutritional composition of the present invention have better results in the areas of memory, in particular short term memory, alertness, learning capacity, attentiveness, concentration capacity and language ability. In particular, children receiving the nutritional composition of the invention have an improved memory if compared to the children of the control group.

These results show that the nutritional composition of the invention is a growing up milk that supports and/or improves the cognitive ability, performance, in particular the learning and memory capacities in children of the age of 1 to 3 years.

These results show that the children receive the nutritional growing up milk have comparable or better changes in the body weight, height and head circumference during development. The growth parameters will also be compared to WHO children growth standard.

These results also show that the children receive the nutritional growing up milk has beneficial gut microbiota and the efficacy from the occurrence of diarrhea.

## Claims

1. The use of a nutritional composition for improving cognitive performance in a child of 1 to 7 years, said nutritional composition comprising:
- one or more protein source,
- one or more source of available carbohydrates,
- one or more lipid source,
- one or more probiotic microorganism, and,
- prebiotics,
wherein at least one of said one or more lipid source comprises DHA (docosahexaenoic acid).

2. The use of claim 1, wherein the child is a child of 1-5, preferably 1-3 years.

3. The use of claim 1 or 2, wherein cognitive performance refers to any one selected from short term memory, long term memory, learning capacity, alertness, attentiveness and concentration capacity.

4. The use of any one of the preceding claims, wherein said nutritional formula comprises added DHA, which is provided in the form of a phospholipids comprising DHA.

5. The use of any one of the preceding claims, wherein said nutritional formula comprises added DHA, which is obtained from egg yolk and/or in which the major form of DHA is phosphatidylcholine (PC) and/or phosphatidylethanolamine (PE).

6. The use of any one of the preceding claims, wherein said nutritional formula comprises at least 10 mg ARA.

7. The use of any one of the preceding claims, wherein said nutritional composition comprises at least 1 g linoleic acid and at least 100 mg α-linolenic acid per 100 g of the dry weight of the nutritional composition.

8. The use of any one of the preceding claims, wherein said nutritional composition comprises, per 100 gram of dry matter, at least 20 mg (milligram) sphingomyelin.

9. The use of any one of the preceding claims, wherein said nutritional composition comprises, per 100 gram of dry matter, at least 40 mg sialic acid.

10. The use of any one of the preceding claims, wherein said nutritional composition comprises, per 100 gram of dry matter, at least 20 mg choline.

11. The use of any one of the preceding claims, wherein said nutritional composition comprises a combination of nutrients, said combination comprising sphingomyelin, sialic acid, choline, thiamine, folic acid, taurine and vitamin D.

12. The use of any one of the preceding claims, wherein the nutritional composition further comprises iron, iodine and zinc.

13. The use of any one of the preceding claims, wherein said protein source provides about 11 to about 18 percent of the total energy, said source of available carbohydrates provides from about 46 to about 54 percent of the total energy, and said lipid source provides from about 31 to about 39 percent of the total energy of the composition.

14. The use of any one of the preceding claims, wherein said nutritional composition comprises at least two different probiotic microorganisms, each of said microorganisms being provided at about 1 x 10⁵ to 1x 10⁹ CFU per g of dry matter of the composition.

15. The use of any one of the preceding claims, wherein said prebiotic comprises one or more selected from inulin, fructooligosaccharides, or a mixture of inulin and fructooligosaccharides.

16. The use of any one of the preceding claims, wherein said nutritional composition is a growing-up milk, which is provided in powdered form.

17. The use of any one of the preceding claims, wherein said nutritional composition comprises the nutrients as detailed in the Tables 1 +4+7.

18. A method for supporting and/improving cognitive performance in a child of 1 to 6 years, said method comprising the step of administrating a nutritional composition comprising:
- one or more protein source,
- one or more source of available carbohydrates,
- one or more lipid source,
- one or more probiotic microorganism, and,
- prebiotics,
wherein said lipid source comprises DHA (docosahexaenoic acid).

19. A method of providing nutrition, the method comprising the step of administering to a child of 1 to 6 years a nutritional composition comprising:
- one or more protein source,
- one or more source of available carbohydrates,
- one or more lipid source,
- one or more probiotic microorganism, and,
- prebiotics,
wherein said lipid source comprises DHA (docosahexaenoic acid).

20. A method for preparing a nutritional composition, said method comprising mixing:
- one or more protein source,
- one or more source of available carbohydrates,
- one or more lipid source,
- one or more probiotic microorganism, and,
- prebiotics,
wherein said lipid source comprises DHA (docosahexaenoic acid), and thereby obtaining the nutritional composition of the invention.

21. The uses and methods according to the preceding claims, wherein said nutritional composition is a growing up milk.

22. The uses and methods according to the preceding claims, wherein said nutritional composition is provided in powdered, reconstitutable form.
